# EUROPEAN PATENT SPECIFICATION

(11) **EP 2 198 052 B1**
(45) Date of publication and mention of the grant of the patent: **17.07.2013**
(21) Application number: 08839452.3
(22) Date of filing: 15.10.2008
(51) Int. Cl.: C12Q 1/68

(54) **METHOD FOR ADMINISTERING ANTICOAGULATION THERAPY**
VERFAHREN ZUR VERABREICHUNG EINER ANTIKOAGULATIONSTHERAPIE
PROCÉDÉ D'ADMINISTRATION D'UN TRAITEMENT ANTICOAGULANT

(30) Priority: 19.10.2007 US 981186 P
(43) Date of publication of application: 23.06.2010
(73) Proprietor: Marshfield Clinic, Marshfield, WI 54449 (US)
(72) Inventor: CALDWELL, Michael, D., Marshfield, WI 54449 (US); BERG, Richard, L., Marshfield, WI 54449 (US); BURMESTER, James, K., Marshfield, WI 54449 (US)
(74) Representative: Duckworth, Timothy John
(86) International application number: PCT/US2008/079966
(87) International publication number: WO 2009/052160

(56) References cited:
- GAGE BRIAN F ET AL: "Use of pharmacogenetics and clinical factors to predict the maintenance dose of warfarin." THROMBOSIS AND HAEMOSTASIS, vol. 91, no. 1, January 2004 (2004-01), pages 87-94, XP002515418 ISSN: 0340-6245 cited in the application
- CALDWELL MICHAEL D ET AL: "Evaluation of genetic factors for warfarin dose prediction." CLINICAL MEDICINE & RESEARCH MAR 2007, vol. 5, no. 1, March 2007 (2007-03), pages 8-16, XP002515419 ISSN: 1539-4182 cited in the application
- RIEDER MARK J ET AL: "Effect of VKORC1 haplotypes on transcriptional regulation and warfarin dose." THE NEW ENGLAND JOURNAL OF MEDICINE 2 JUN 2005, vol. 352, no. 22, 2 June 2005 (2005-06-02), pages 2285-2293, XP002515420 ISSN: 1533-4406 cited in the application
- STEC DAVID E ET AL: "Functional polymorphism in human CYP4F2 decreases 20-HETE production" PHYSIOLOGICAL GENOMICS, vol. 30, no. 1, June 2007 (2007-06), pages 74-81, XP002515421 ISSN: 1094-8341 cited in the application
- CALDWELL MICHAEL D ET AL: "CYP4F2 genetic variant alters required warfarin dose" BLOOD, vol. 111, no. 8, April 2008 (2008-04), pages 4106-4112, XP002515422 ISSN: 0006-4971
- PEREZ-ANDREU V ET AL: "Pharmacogenetic relevance of CYP4F2 V433M polymorphism on acenocoumarol therapy", BLOOD 2009 AMERICAN SOCIETY OF HEMATOLOGY USA, vol. 113, no. 20, 2009, pages 4977-4979, ISSN: 0006-4971
- MCDONALD M G ET AL: "CYP4F2 is a vitamin K1 oxidase: An explanation for altered warfarin dose in carriers of the V433M variant", MOLECULAR PHARMACOLOGY 2009 AMERICAN SOCIETY FOR PHARMACOLOGY AND EXPERIMENTAL THERAPY USA, vol. 75, no. 6, 2009, pages 1337-1346, ISSN: 0026-895X
- 'Cytochrome P450 family', [Online] Retrieved from the Internet: <URL:http://www.genenames.org/genefamily/cy p.php> [retrieved on 2010-07-01]

## Description

This non-provisional application claims the benefit of U.S. Provisional application 60/981,186, filed October 19, 2007.

### STATEMENT AS TO RIGHTS TO INVENTIONS MADE UNDER FEDERALLY SPONSORED RESEARCH AND DEVELOPMENT

Not applicable.

### FIELD OF THE INVENTION

The present invention relates to a method for treating a patient with an anticoagulant to optimize drug therapy and to prevent an adverse drug response. More particularly, the present invention relates to a method and system for use in treating a patient with Coumadin^{®} or a substance containing warfarin. The present invention utilizes specific genetic markers to determine the effectiveness of the dosing regimen and, if necessary, to suggest a new more optimal drug dose.

### BACKGROUND OF THE INVENTION

One of the goals in the emerging fields of pharmacogenetics and personalized medicine is to use genotypic data to reduce adverse drug reactions (ADRs) and select 'the right medicine, for the right patient, at the right dose'. Anticoagulant drugs, such as warfarin, are commonly prescribed to patients to prevent blood clotting. However, anticoagulant drugs have also been identified as one of the more dangerous of medications. For example, approximately 700,000 patients with atrial fibrillation receive daily warfarin in the USA, resulting in 17,000 major bleeds, of which about 4,000 are fatal.

Substantial inter-patient variability exists in the anti-thrombotic response to warfarin. Consequently, warfarin therapy requires intensive monitoring via the International Normalized Ratio (INR) to guide its dosing, to maintain a therapeutic level of anti-thrombosis and to minimize the risk of bleeding

Currently, coumarin-based anticoagulant drugs are the definitive treatment worldwide for the long-term prevention of thromboembolic events. For example, in 2003, it is believed that a total of about 21.2 million prescriptions were written for the oral anticoagulant warfarin (a derivative of coumarin) in the United States alone. However, management of warfarin therapy is challenging in two respects: first, a safe and effective stabilization dose must be determined during the early months of therapy, and second, maintenance doses must be adjusted to compensate for changes in patients' weight, diet, disease state, and concomitant use of other medications.

S-warfarin is primarily metabolized by cytochrome P450 2C9 (CYP2C9). Single nucleotide polymorphisms in the CYP2C9 gene correlate with reduced enzymatic efficiency. This enzymatic inefficiency leads to a reduction in the dose of warfarin required to achieve a stable international normalization ratio (INR) for prothrombin time. Once the effects of these genetic variants were understood, it was proposed that the use of genotype would identify those patients susceptible to ADRs. However, it has been shown that, although useful, genotyping or haplotyping for CYP2C9 alone was insufficient to avoid all ADRs related to warfarin therapy.

Accordingly, there is a need for a method to more accurately determine warfarin dosage requirement for each individual.

### SUMMARY OF THE INVENTION

The present invention provides a method for optimising an anticoagulant dosage for a patient requiring the anticoagulant, wherein the anticoagulant is a coumarin based anticoagulant, said method comprising:
determining the CYP4F2 genotype of the patient; and
determining the anticoagulant dosage for the patient as a function of variables, wherein at least one variable comprises the CYP4F2 genotype.

In one specific embodiment, the method determines the initial anticoagulant dosage for the patient. In another embodiment, the method further comprises calculating a new anticoagulant dose regimen.

In another embodiment, the variables further comprise the patient's age, gender, body surface area, diabetic condition, presence of artificial heart valve, or a combination thereof. In one specific embodiment, the variables further comprise the patient's CYP2C9 and/or VKORC1 genotype.

In another embodiment, the anticoagulant is Coumadin^{®}.

In another embodiment, in the method of calculating an anticoagulant dosage for a patient requiring the anticoagulant the CYP4F2 genotype is deduced through analysis of rs2108622. In other aspects, the CYP4F2 genotype is deduced through analysis of a SNP in linkage disequilibrium with rs2108622, preferably a SNP selected from the group of rs3093114, rs3093106 and rs3093105.

### BRIEF DESCRIPTION OF THE DRAWINGS

Figure 1. Correlation of rs2108622 with rs3093114, r 3093106, and rs3093105. These three genotypes associate with stable therapeutic warfarin dose and are in linkage disequilibrium with rs210862.

Figure 2. Depiction of relative statistical relationships of 1228 SNPs with warfarin therapeutic dose, Marshfield model. The P-value (log scale) for each polymorphism relative to warfarin dose was plotted. Dashed line shows the adjusted threshold for significance.

Figure 3. Median warfarin therapeutic dose by CYP4F2 genotype and study site. Box plots of dose by site and CYP4F2. All cases. Boxes extend from the 25^{th} to the 75^{th} percentiles, with a horizontal line at the median and vertical lines extending to the 10^{th} and 90^{th} percentiles.

Figure 4. Marshfield model residuals by CYP4F2 genotype and study site (* 1 genotypes only). Model adjusts for age, body surface area, VKORC1, CYP2C9. Boxes extend from the 25^{th} to the 75^{th} percentiles, with a horizontal line at the median and vertical lines extending to the 10^{th} and 90^{th} percentiles.

### DETAILED DESCRIPTION OF THE INVENTION

### In General

Before the present materials and methods are described, it is understood that this invention is not limited to the particular methodology, protocols, materials, and reagents described, as these may vary. It is also to be understood that the terminology used herein is for the purpose of describing particular embodiments only, and is not intended to limit the scope of the present invention which will be limited only by the appended claims.

As used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", and "having" can be used interchangeably.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are now described. All references cited in this specification are to be taken as indicative of the level of skill in the art. Nothing herein is to be construed as an admission that the invention is not entitled to antedate such disclosure by virtue of prior invention.

The practice of the present invention will employ, unless otherwise indicated, conventional techniques of molecular biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Pat. No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); and Handbook Of Experimental Immunology, Volumes I-IV (D. M. Weir and C. C. Blackwell, eds., 1986).

In describing the present invention, the following terms will be employed and are intended to be defined as indicated below.

The term "anticoagulant" as used herein includes, but is not limited to, warfarin and warfarin derivatives such as Coumadin®, warfarin sodium salt, and coumarin derivatives. Other common warfarin brands are Panwarfin® and Sofarin®. Commonly used experimental warfarin derivatives are 6-, 7-, 8-, 4'-hdroxy, 6-chloro- and 6-bromowarfarin, 3, 4-dihydro-2*H*-pyran ketals, acyl and aryl warfarin derivatives, and 4-Hydrosy-3-[1-(4-chlorophenl)-3-oxobutyl]-2*H-*1-benzopyran-2-one, oxime. In the invention the anti-coagulant is a coumarin based anti-coagulant.

Furthermore, wherever the generic term "anticoagulant" is used herein it is also intended to mean species which employ any or more of the individual anticoagulants as defined and/or alluded to hereinabove.

A "single nucleotide polymorphism" or "SNP" refers to a variation in the nucleotide sequence of a polynucleotide that differs from another polynucleotide by a single nucleotide difference. For example, without limitation, exchanging one A for one C, G or T in the entire sequence of polynucleotide constitutes a SNP. It is possible to have more than one SNP in a particular polynucleotide. For example, at one position in a polynucleotide, a C may be exchanged for a T, at another position a G may be exchanged for an A and so on. When referring to SNPs, the polynucleotide is most often DNA. The term "allele" refers to one or more alternative forms of a particular sequence that contains a SNP. The sequence may or may not be within a gene.

"Amplification" refers to any means by which a polynucleotide sequence is copied and thus expanded into a larger number of polynucleotide sequences, e.g., by reverse transcription, polymerase chain reaction or ligase chain reaction, among others.

An "isolated" polynucleotide or polypeptide is one that is substantially pure of the materials with which it is associated in its native environment. By substantially free, is meant at least 50%, at least 55%, at least 60%, at least 65%, at advantageously at least 70%, at least 75%, more advantageously at least 80%, at least 85%, even more advantageously at least 90%, at least 91%, at least 92%, at least 93%, at least 94%, at least 95%, at least 96%, at least 97%, most advantageously at least 98%, at least 99%, at least 99.5%, at least 99.9% free of these materials.

In the context of the present invention, the following abbreviations for the commonly occurring nucleic acid bases are used. "A" refers to adenosine, "C" refers to cytidine, "G" refers to guanosine, "T" refers to thymidine, and "U" refers to uridine.

The term "nucleic acid" typically refers to large polynucleotides. A "polynucleotide" means a single strand or parallel and anti-parallel strands of a nucleic acid. Thus, a polynucleotide may be either a single-stranded or a double-stranded nucleic acid. A polynucleotide is not defined by length and thus includes very large nucleic acids, as well as short ones, such as an oligonucleotide The term "oligonucleotide" typically refers to short polynucleotides, generally no greater than about 50 nucleotides. It will be understood that when a nucleotide sequence is represented by a DNA sequence (i.e., A, T, G, C), this also includes an RNA sequence (i.e., A, U, G, C) in which "U" replaces "T."

Conventional notation is used herein to describe polynucleotide sequences: the left-hand end of a single-stranded polynucleotide sequence is the 5' -end; the left-hand direction of a double-stranded polynucleotide sequence is referred to as the 5' -direction. The direction of 5' to 3' addition of nucleotides to nascent RNA transcripts is referred to as the transcription direction. The DNA strand having the same sequence as an mRNA is referred to as the "coding strand". Sequences on a DNA strand which are located 5' to a reference point on the DNA are referred to as "upstream sequences". Sequences on a DNA strand which are 3' to a reference point on the DNA are referred to as "downstream sequences."

"Primer" refers to a polynucleotide that is capable of specifically hybridizing to a designated polynucleotide template and providing a point of initiation for synthesis of a complementary polynucleotide. Such synthesis occurs when the polynucleotide primer is placed under conditions in which synthesis is induced, i.e., in the presence of nucleotides, a complementary polynucleotide template, and an agent for polymerization such as DNA polymerase.

"Probe" refers to a polynucleotide that is capable of specifically hybridizing to a designated sequence of another polynucleotide. "Probe" as used herein encompasses oligonucleotide probes. A probe may or may not provide a point of initiation for synthesis of a complementary polynucleotide. A probe specifically hybridizes to a target complementary polynucleotide, but need not reflect the exact complementary sequence of the template. In such a case, specific hybridization of the probe to the target depends on the stringency of the hybridization conditions. For use in SNP detection, some probes are allele-specific, and hybridization conditions are selected such that the probe binds only to a specific SNP allele. Probes can be labeled with, e.g., detectable moieties, such as chromogenic, radioactive or fluorescent moieties, and used as detectable agents.

As used herein, "label" refers to a group covalently attached to a polynucleotide. The label may be attached anywhere on the polynucleotide but is preferably attached at one or both termini of the polynucleotide. The label is capable of conducting a function such as giving a signal for detection of the molecule by such means as fluorescence, chemiluminescence, and electrochemical luminescence. Alternatively, the label allows for separation or immobilization of the molecule by a specific or non-specific capture method.

The term "capable of hybridizing under stringent conditions" as used herein refers to annealing a first nucleic acid to a second nucleic acid under stringent conditions as defined herein. Stringent hybridization conditions typically permit the hybridization of nucleic acid molecules having at least 70% nucleic acid sequence identity with the nucleic acid molecule being used as a probe in the hybridization reaction. For example, the first nucleic acid may be a test sample or probe, and the second nucleic acid may be the sense or antisense strand of a nucleic acid or a fragment thereof. Hybridization of the first and second nucleic acids may be conducted under stringent conditions, e.g., high temperature and/or low salt content that tend to disfavor hybridization of dissimilar nucleotide sequences. Alternatively, hybridization of the first and second nucleic acid may be conducted under reduced stringency conditions, e.g. low temperature and/or high salt content that tend to favor hybridization of dissimilar nucleotide sequences. Low stringency hybridization conditions may be followed by high stringency conditions or intermediate medium stringency conditions to increase the selectivity of the binding of the first and second nucleic acids. The hybridization conditions may further include reagents such as, but not limited to, dimethyl sulfoxide (DMSO) or formamide to disfavor still further the hybridization of dissimilar nucleotide sequences. A suitable hybridization protocol may, for example, involve hybridization in 6xSSC (wherein 1x SSC comprises 0.015 M sodium citrate and 0.15 M sodium chloride), at 65 degrees Celsius in an aqueous solution, followed by washing with 1xSSC at 65 degrees C. Formulae to calculate appropriate hybridization and wash conditions to achieve hybridization permitting 30% or less mismatch between two nucleic acid molecules are disclosed, for example, in Meinkoth et al. (1984) Anal. Biochem. 138: 267-284; the content of which is herein incorporated by reference in its entirety. Protocols for hybridization techniques are well known to those of skill in the art and standard molecular biology manuals may be consulted to select a suitable hybridization protocol without undue experimentation. See, for example, Sambrook et al. (2001) Molecular Cloning: A Laboratory Manual, 3rd ed., Cold Spring Harbor Press, the contents of which are herein incorporated by reference in their entirety. Low, intermediate and high stringency hybridization conditions are described in more detail below.

The term "associated with" or "associated" in the context of this invention refers to, e.g., a nucleic acid and a phenotypic trait, that are in linkage disequilibrium, i.e., the nucleic acid and the trait are found together in progeny more often than if the nucleic acid and trait segregated separately.

The term "linkage disequilibrium" refers to a non-random segregation of genetic loci. This implies that such loci are in sufficient physical proximity along a length of a chromosome that they tend to segregate together with greater than random frequency.

The term "genetically linked" refers to genetic loci that are in linkage disequilibrium and statistically determined not to assort independently. Genetically linked loci assort dependently from 51 % to 99% of the time or any whole number value there between, preferably at least 60%, 70%, 80%, 90%, 95% or 99%. The term "proximal" means genetically linked when used in the context of genetic loci.

The term "marker" or "molecular marker" refers to a genetic locus (a "marker locus") used as a point of reference when identifying genetically linked loci. The term also refers to nucleic acid sequences complementary to the genomic sequences, such as nucleic acids used as probes.

The term "interval" refers to a continuous linear span of chromosomal DNA with termini defined by and including molecular markers.

The terms "nucleic acid," "polynucleotide," "polynucleotide sequence" and "nucleic acid sequence" refer to single-stranded or double-stranded deoxyribonucleotide or ribonucleotide polymers, or chimeras thereof. As used herein, the term can additionally or alternatively include analogs of naturally occurring nucleotides having the essential nature of natural nucleotides in that they hybridize to single-stranded nucleic acids in a manner similar to naturally occurring nucleotides (e.g., peptide nucleic acids). Unless otherwise indicated, a particular nucleic acid sequence of this invention optionally encompasses complementary sequences, in addition to the sequence explicitly indicated. The term "gene" is used to refer to, e.g., a cDNA and an mRNA encoded by the genomic sequence, as well as to that genomic sequence,.

The term "homologous" refers to nucleic acid sequences that are derived from a common ancestral gene through natural or artificial processes (e.g., are members of the same gene family), and thus, typically, share sequence similarity. Typically, homologous nucleic acids have sufficient sequence identity that one of the sequences or its complement is able to selectively hybridize to the other under selective hybridization conditions. The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences have about at least 80% sequence identity, preferably at least 90% sequence identity, and most preferably 95%, 97%, 99%, or 100% sequence identity with each other. A nucleic acid that exhibits at least some degree of homology to a reference nucleic acid can be unique or identical to the reference nucleic acid or its complementary sequence.

The term "isolated" refers to material, such as a nucleic acid or a protein, which is substantially free from components that normally accompany or interact with it in its naturally occurring environment. The isolated material optionally comprises material not found with the material in its natural environment, e.g., a cell. In addition, if the material is in its natural environment, such as a cell, the material has been placed at a location in the cell (e.g., genome or subcellular organelle) not native to a material found in that environment. For example, a naturally occurring nucleic acid (e.g., a promoter) is considered to be isolated if it is introduced by non-naturally occurring means to a locus of the genome not native to that nucleic acid. Nucleic acids which are "isolated" as defined herein, are also referred to as "heterologous" nucleic acids.

The term "recombinant" indicates that the material (e.g., a nucleic acid or protein) has been synthetically (non-naturally) altered by human intervention. The alteration to yield the synthetic material can be performed on the material within or removed from its natural environment or state. For example, a naturally occurring nucleic acid is considered a recombinant nucleic acid if it is altered, or if it is transcribed from DNA which has been altered, by means of human intervention performed within the cell from which it originates. See, e.g., Compounds and Methods for Site Directed Mutagenesis in Eukaryotic Cells, Kmiec, U.S. Pat. No. 5,565,350; In Vivo Homologous Sequence Targeting in Eukaryotic Cells; Zarling et al., PCT/US93/03868.

When a patient begins taking an anticoagulant or any medication for a length of time, a titration of the amount of drug taken by the patient is necessary in order to achieve the optimal benefit of the drug and, at the same time, to prevent any undesirable side effects that taking too much of the drug could produce. Thus, there is a continuous balance between taking enough drug in order to gain the benefits from that drug and at the same time not taking so much drug as to illicit a toxic event.

There is large inter-individual variability in the patient pharmocodynamic and pharmacokinetic interactions of drugs. What may be an appropriate drug dose for one individual, may be too much or too little for another. Prior to this invention a physician was required to estimate the correct drug dosage for a patient and then to experiment with that dosage, usually by trial and error, until the correct dosage was achieved. Likewise, the FDA labeling of a drug suggests dosages based on epidemiological studies and again does not account for inter-individual variability.

The invention provides a method for optimising an anticoagulant dosage for a patient requiring the anticoagulant, wherein the anticoagulant is a coumarin based anticoagulant, said method comprising:
determining the CYP4F2 genotype of the patient; and
determining the anticoagulant dosage for the patient as a function of variables, wherein at least one variable comprises the CYP4F2 genotype. The method may prevent an adverse drug response. The method can be used to determine an initial anticoagulant dosage and/or a prospective anticoagulation dosage.

Typically, a physician prescribes an anticoagulant for a patient based on the FDA recommended dose on the label of the anticoagulant. The physician then re-evaluates the patient, usually daily, either in person or remotely depending on the agent being prescribed. During the subsequent evaluations by the physician, INR tests are monitored and sequentially compared to determine if there are any toxicities associated with the anticoagulant and/or to see if the desired effect of the anticoagulant is being achieved. Based on this evaluation by the physician and the current anticoagulant dose, new anticoagulant dose is calculated.

In the examples below, Applicants have determined that a SNP found within the CYP4F2 gene at position rs2108622 associates with a highly significant difference in warfarin dose, with those patients who are homozygous CC requiring less warfarin than predicted and those with T alleles require more warfarin than predicted. As used herein, the CC/TT designation refers to the opposite strand of DNA from the coding strand of DNA which is GG/AA.

In one particular aspect, methods of the invention determine the patient's initial anticoagulation dosage as a function of the patient's CYP4F2 genotype, as well as preferably considering variables comprising patient's CYP2C9 genotype and VKORC1 genotype information. As described more fully below, it should be appreciated that there are a variety of methods known in the art for determining such genotypes. For example, genotypes can be directly determined by analyzing individual SNPs within that gene or can be inferred by analyzing the haplotype of that gene. Still alternatively, genotypes can be determined by analyzing the haplotype of a gene that is closely associated with the gene of interest. Such methods are well known in the art and the scope of present invention includes both direct and indirect methods of determining CYP4F2, CYP2C9 and VKORC1 genotypes. Haplotypes for CYP2C9 and VKORC1 are well known to one skilled in the art. See, for example, Aithal et al., Lancet, 1999, 353, 717-719 for CYP2C9 haplotypes and Reider et al., N. Engl J Med, 2005, 352, 2285-2293 for VKORC1 haplotypes. Haplotypes compiled across the human genome are also available at various internet accessible databases, e.g., www.hapmap.org.

Some aspects of the invention utilize relevant genotypic, clinical, and environmental data to determine anticoagulant dosage. In some embodiments, additional information such as current dose and INR data are included to determine prospective dosage requirement. For example, in some embodiments, the variables used to determine initial anticoagulation dosage further comprise the patient's age, gender, body surface area, diabetic condition, presence of artificial heart valve, or a combination thereof.

In our Examples below, we provide a description of our methodology used to identify SNPs that predict warfarin dose response. Second, we provide a brief summary of the results with respect to rs2108622. Third, we provide figures which illustrate the results. Finally, we provide a brief summary of a small internal confirmatory study in which 61 new cases were tested.

Table 1, below, discloses the correlation between rs2108622 status and warfarin dosage.

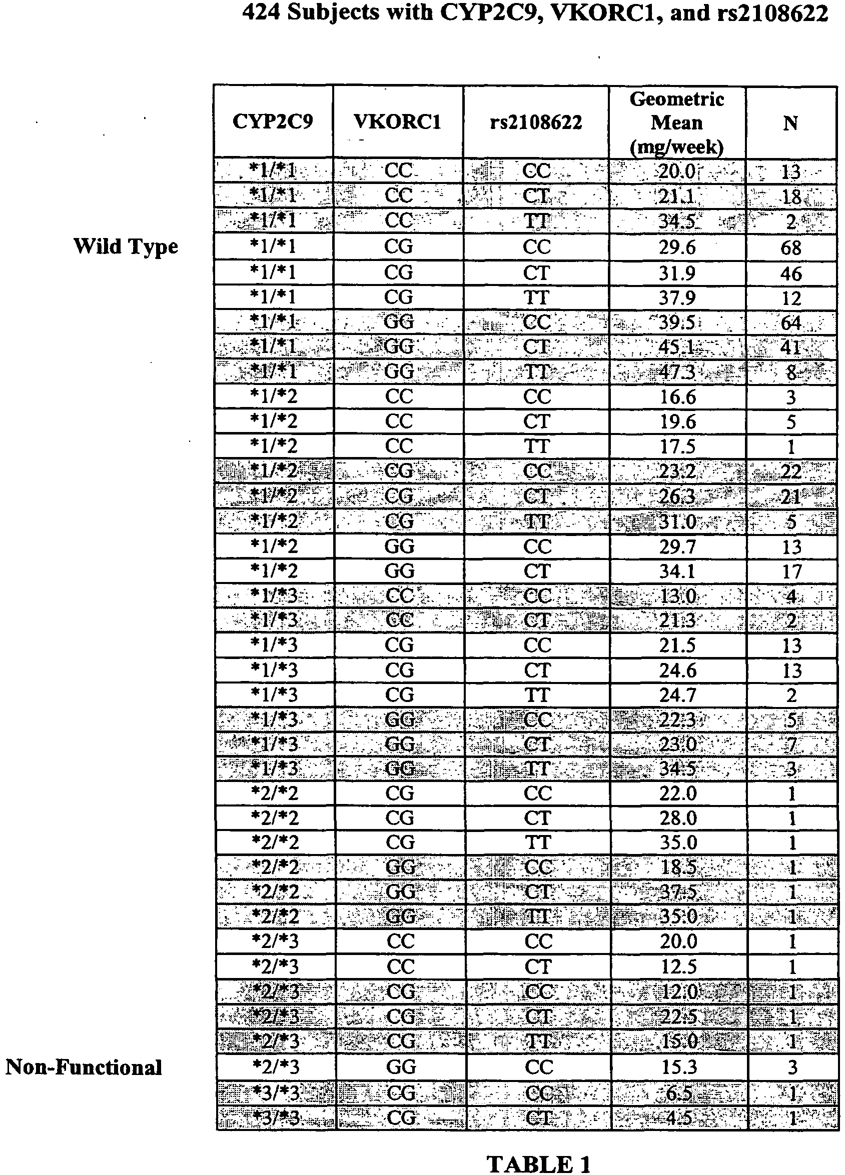

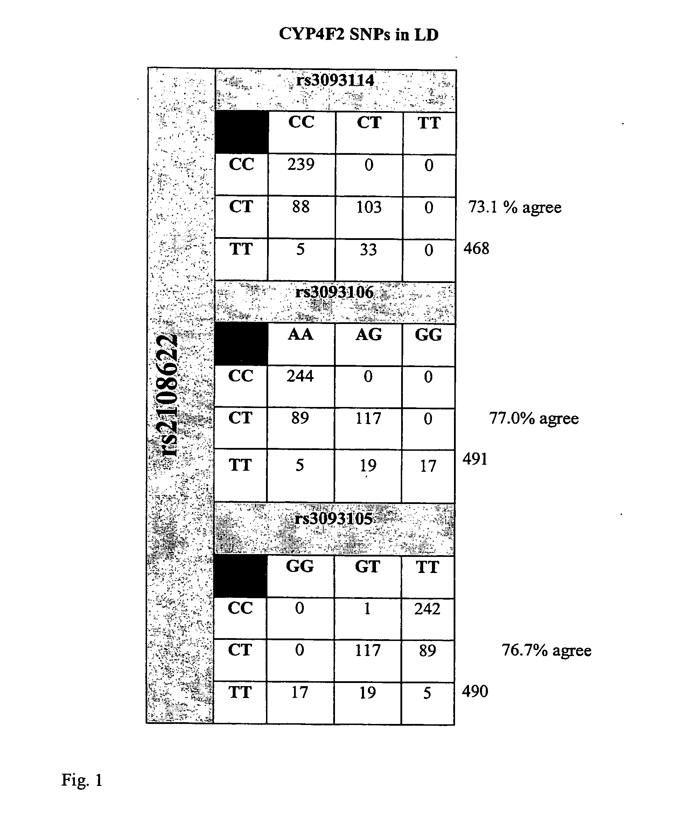

Additionally we have found rs3093114, rs3093106, and rs3093105 to significantly associate with stable therapeutic warfarin dose and to be in LD with rs210862. Therefore, one of skill in the art would understand that evaluation of a patients genotype for rs3093114, rs3093106 and rs3093105 would be correct about 73.1%, 77.0% or 76.7% of the time, respectively for evaluating the rs210862 status. The correlation of rs2108622 with the three SNPs is illustrated in Figure 1.

### Identifying SNPs of the Invention

In another aspect, the present invention is directed to the identification of one or more SNPs in a biological sample obtained from an individual.

### A. Biological Sample

Biological samples useful in the practice of the methods of the invention can be any biological sample from which any of genomic DNA, mRNA, unprocessed RNA transcripts of genomic DNA or combinations of the three can be isolated. As used herein, "unprocessed RNA" refers to RNA transcripts which have not been spliced and therefore contain at least one intron. Suitable biological samples are removed from human patient and include, but are not limited to, blood, buccal swabs, hair, bone, and tissue samples, such as skin or biopsy samples. Biological samples also include cell cultures established from an individual.

Genomic DNA, mRNA, and/or unprocessed RNA transcripts are isolated from the biological sample by conventional means known to the skilled artisan. See, for instance, Sambrook et al. (2001, Molecular Cloning: A Laboratory Manual, Cold Spring Harbor Laboratory Press, Cold Spring Harbor, N.Y.) and Ausubel et al. (eds., 1997, Current Protocols in Molecular Biology, John Wiley & Sons, New York). The isolated genomic DNA, mRNA, and/or unprocessed RNA transcripts is used, with or without amplification, to detect a SNP of the invention.

### B. Amplification

Many SNP identification methods that can be used in the methods of the invention involve amplifying a target polynucleotide sequence prior to detecting the SNP identity. A "target polynucleotide sequence" is a region of the genomic DNA, mRNA or unprocessed RNA containing the SNP of interest. Some methods, including the 5' nuclease assay described herein, combine the amplification and detection processes in one step, as described elsewhere herein. Other methods, such as the invasive cleavage assay also described herein, use signal amplification and are thereby sufficiently sensitive such that the genomic nucleic acid sample does not need to be amplified.

Amplification of a target polynucleotide sequence may be carried out by any method known to the skilled artisan. See, for instance, Kwoh et al., (1990, Am. Biotechnol. Lab. 8, 14-25) and Hagen-Mann, et al., (1995, Exp. Clin. Endocrinol. Diabetes 103:150-155). Amplification methods include, but are not limited to, polymerase chain reaction ("PCR") including RT-PCR, strand displacement amplification (Walker et al., 1992, PNAS 89, 392-396; Walker et al., 1992, Nucleic Acids Res. 20, 1691-1696), strand displacement amplification using Phi29 DNA polymerase (U.S. patent 5,001,050), transcription-based amplification (Kwoh et al., 1989, PNAS 86, 1173-1177), self-sustained sequence replication ("3SR") (Guatelli et al., 1990, PNAS 87, 1874-1878; Mueller et al., 1997, Histochem. Cell Biol. 108:431-437), the Q.beta. replicase system (Lizardi et al., 1988, BioTechnology 6, 1197-1202; Cahill et al., 1991, Clin., Chem. 37:1482-1485), nucleic acid sequence-based amplification ("NASBA") (Lewis, 1992, Genetic Engineering News 12 (9), 1), the repair chain reaction ("RCR") (Lewis, 1992, supra), and boomerang DNA amplification (or "BDA") (Lewis, 1992, supra). PCR is the preferred method of amplifying the target polynucleotide sequence.

PCR may be carried out in accordance with known techniques. See, e.g., Bartlett et al., eds., 2003, PCR Protocols Second Edition, Humana Press, Totowa, N.J. and U.S. Pat. Nos. 4,683,195; 4,683,202; 4,800,159; and 4,965,188. In general, PCR involves, first, treating a nucleic acid sample (e.g., in the presence of a heat stable DNA polymerase) with a pair of amplification primers. One primer of the pair hybridizes to one strand of a target polynucleotide sequence. The second primer of the pair hybridizes to the other, complementary strand of the target polynucleotide sequence. The primers are hybridized to their target polynucleotide sequence strands under conditions such that an extension product of each primer is synthesized which is complementary to each nucleic acid strand. The extension product synthesized from each primer, when it is separated from its complement, can serve as a template for synthesis of the extension product of the other primer. After primer extension, the sample is treated to denaturing conditions to separate the primer extension products from their templates. These steps are cyclically repeated until the desired degree of amplification is obtained. The amplified target polynucleotide may be used in one of the detection assays described elsewhere herein to identify the SNP present in the amplified target polynucleotide sequence.

### C. Oligonucleotide Primers and Probes

Nucleic acid amplification techniques, such as the foregoing, and SNP allele detection methods, as described below, may involve the use of a primer, a pair of primers, or two pairs of primers which specifically bind to nucleic acid containing the SNP to be detected, and do not bind to nucleic acid that does not contain the SNP to be detected under the same hybridization conditions. Such probes are sometimes referred to as "amplification primers" herein.

In some detection assays, a polynucleotide probe, which is used to detect DNA containing a SNP of interest, is a probe which binds to DNA encoding a specific SNP allele, but does not bind to DNA that does not encode that specific SNP allele under the same hybridization conditions. For instance, the detection probe used for 5' nuclease assay, described herein, straddles a SNP site and discriminates between alleles. In other assays, a polynucleotide probe which is used to detect DNA containing a SNP of interest is a probe that binds to either SNP allele at a sequence that does not include the SNP. This type of probe may bind to a sequence immediately 3' to the SNP or may bind to a sequence that is 3' to the SNP and removed from the SNP by one or more bases. In some cases, the polynucleotide probe is labeled with one or more labels, such as those, for instance, set forth elsewhere herein in the 5' nuclease assay.

Probes and primers may be any suitable length, but are typically oligonucleotides from 5, 6, 8 or 12 nucleotides in length up to 40, 50 or 60 nucleotides in length, or more. The oligonucleotides typically comprises a region of nucleotide sequence that hybridizes under stringent conditions to at least about 5, 6, 8, 12, 20, 25, 40, 50 or more consecutive nucleotides in the target polynucleotide sequence. The skilled artisan knows where the region of consecutive nucleotides intended to hybridize to the target polynucleotide sequence must be located in the oligonucleotide, based on the intended use of the oligonucleotide. For instance, in an oligonucleotide for use in a primer extension assay, the skilled artisan knows the region of consecutive nucleotides must include the 3' terminal nucleotide. The probes and primers are typically substantially purified. Such probes and/or primers may be immobilized on or coupled to a solid support such as a bead, glass slide or chip in accordance with known techniques, and/or coupled to or labelled with a detectable label such as a fluorescent compound, a chemiluminescent compound, a radioactive element, or an enzyme in accordance with known techniques.

Probes and primers are designed using the sequences flanking the SNP in the target polynucleotide sequence. Depending on the particular SNP identification protocol utilized, the consecutive nucleotides of the region that hybridizes to a target polynucleotide sequence may include the target SNP position. Alternatively the region of consecutive nucleotides may be complementary to a sequence in close enough proximity 5' and/or 3' to the SNP position to carry out the desired assay.
The skilled artisan can readily design primer and probe sequences using the sequences provided herein. Considerations for primer and probe design with regard to, for instance, melting temperature and avoidance of primer-dimers, are well known to the skilled artisan. In addition, a number of computer programs, such as Primer Express. (Applied Biosystems, Foster City, Calif.) and Primo SNP 3.4 (Chang Bioscience, Castro Valley, Calif.), can be readily used to obtain optimal primer/probe sets. The probes and primers may be chemically synthesized using commercially available reagents and synthesizers by methods that are well-known in the art (see, e.g., Herdwijn, 2004, Oligonucleotide Synthesis: Methods and Applications, Humana Press, Totowa, N.J.).

### D. Methods of Identifying SNP Alleles

The process of identifying the nucleotide present at one or more of the SNP positions disclosed and claimed herein is referred to herein by phrases including, but not limited to: "SNP identification", "SNP genotyping", "SNP typing", "SNP detection" and "SNP scoring".

The method of the invention can identify a nucleotide occurrence for either the plus or minus strand of DNA. That is, the invention encompasses not only identifying the nucleotide at the SNP position in the strand, but also identifying the nucleotide at the SNP position in the corresponding complementary minus strand. For instance, for a SNP in which the allele associated with an elevated risk of a disease or malady has a "C" at the SNP on the plus strand, detecting a "G" in the SNP position of the complementary, minus strand is also indicative of that same elevated risk of disease or malady.

There are numerous methods of SNP identification known to the skilled artisan. See, for instance, Kwok (2001, Annu. Rev. Genomics Hum. Genet. 2:235-258) and Theophilus et al., (2002, PCR Mutation Detection Protocols, Humana Press, Totowa, N.J.). Any may be used in the practice of the present invention. SNP identification methods include, but are not limited to, 5' nuclease assay, primer extension or elongation assays, allele specific oligonucleotide ligation, allele specific hybridization, sequencing, invasive cleavage reaction, branch migration assay, single strand conformational polymorphism (SSCP), denaturing gradient gel electrophoresis (DGGE) and immunoassay. Many of these assays have or can be adapted for microarrays. See, for instance, Erdogan et al. (2001, Nuc. Acids Res. 29:e36); O'Meara et al. (2002, Nuc. Acids Res. 30:e75); Pastinen et al. (1997, Genome Res. 7:606-614); Pastinen et al. (2000, Genome Res. 10:1031-1042); and U.S. Pat. No. 6,294,336. Preferred SNP genotyping methods are the 5' nuclease assay, primer extension assays and sequencing.

The 5' nuclease assay, also known as the 5' nuclease PCR assay and the TaqMan Assay (Applied Biosystems, Foster City, Calif.), provides a sensitive and rapid means of genotyping SNPs. The 5' nuclease assay detects, by means of a probe, the accumulation of a specific amplified product during PCR. The probe is designed to straddle a target SNP position and hybridize to the target polynucleotide sequence containing the SNP position only if a particular SNP allele is present. During the PCR reaction, the DNA polymerase, which extends an amplification primer annealed to the same strand and upstream of the hybridized probe, uses its 5' nuclease activity and cleaves the hybridized probe. There are different ways to detect the probe cleavage. In one common variation, the 5' nuclease assay utilizes an oligonucleotide probe labeled with a fluorescent reporter dye at the 5' end of the probe and a quencher dye at the 3' end of the probe. See, for instance, Lee et al., (1993), Nuc. Acids Res. 21:3761-3766), Livak (1999, Genet. Anal. 14:143-149) and U.S. Pat. Nos. 5,538,848, 5,876,930, 6,030,787, 6,258,569 and 6,821,727. The proximity of the quencher dye to the fluorescent reporter in the intact probe maintains a reduced fluorescence for the reporter. Cleavage of the probe separates the fluorescent reporter dye and the quencher dye, resulting in increased fluorescence of the reporter. The 5' nuclease activity of DNA polymerase cleaves the probe between the reporter and the quencher only if the probe hybridizes to the target, and the target is amplified during PCR. Accumulation of a particular PCR product is thus detected directly by monitoring the increase in fluorescence of the reporter dye. In another variation, the oligonucleotide probe for each SNP allele has a unique fluorescent dye and detection is by means of fluorescence polarization (Kwok, 2002, Human Mutat. 19:315-323). This assay advantageously can detect heterozygotes.

The primer extension reaction (also called "mini-sequencing", "single base extension assay" or "single nucleotide extension assay", and "primer elongation assay") involves designing and annealing a primer to a sequence downstream of a target SNP position in an amplified target polynucleotide sequence ("amplified target"). A mix of dideoxynucleotide triphosphates (ddNTPs) and/or deoxynucleotide triphosphates (dNTPs) are added to a reaction mixture containing amplified target, primer, and DNA polymerase. Extension of the primer terminates at the first position in the PCR amplified target where a nucleotide complementary to one of the ddNTPs in the mix occurs. The primer can be annealed to a sequence either immediately 3' to or several nucleotides removed from the SNP position. For single base or single nucleotide extension assays, the primer is annealed to a sequence immediately 3' the SNP position. If the primer anneals to a sequence several nucleotides removed from the target SNP, the only limitation is that the template sequence between the 3' end of the primer and the SNP position can not contain a nucleotide of the same type as the one to be detected, or this will cause premature termination of the extension primer. Alternatively, if all four ddNTPs alone, and no dNTPs, are added to the reaction mixture, the primer will always be extended by only one nucleotide, corresponding to the target SNP position. In this instance, primers are designed to bind to a sequence one nucleotide downstream from the SNP position. In other words, the nucleotide at the 3' end of the primer hybridizes to the nucleotide immediately 3' to the SNP position. Thus, the first nucleotide added to the primer is at the SNP. In one common variation, the ddNTPs used in the assay each have a unique fluorescent label, enabling the detection of the specific nucleotide added to the primer. SNaPshot from Applied Biosystems is a commercially available kit for single nucleotide primer extension using fluorescent ddNTPs, and can be multiplexed. SNP-IT.TM. (Orchid Cellmark, Princeton, N.J.) is another commercially available product using a primer extension assay for identifying SNPs (see also U.S. Pat. No. 5,888,819). Some variations of the primer extension assay can identify heterozygotes.

An alternate detection method uses mass spectrometry to detect the specific nucleotide added to the primer in a primer extension assay. See, for instance, Haffet al. (1997, Genome Res. 7:378-388). Mass spectrometry ("mass spec") takes advantage of the unique mass of each of the four nucleotides of DNA. SNPs can be unambiguously genotyped based on the slight differences in mass, and the corresponding time of flight differences, inherent in nucleic acid molecules having different nucleotides at a single base position. MALDI-TOF (Matrix Assisted Laser Desorption Ionization-Time of Flight) mass spectrometry technology is preferred for extremely precise determinations of molecular mass, such as SNPs. Numerous approaches to SNP analysis have been developed based on mass spectrometry.

For detection by mass spectrometry, extension by only one nucleotide is preferable, as it minimizes the overall mass of the extended primer, thereby increasing the resolution of mass differences between alternative SNP nucleotides. Furthermore, mass-tagged dideoxynucleoside triphosphates (ddNTPs) can be employed in the primer extension reactions in place of unmodified ddNTPs. This increases the mass difference between primers extended with these ddNTPs, thereby providing increased sensitivity and accuracy, and is particularly useful for typing heterozygous base positions. Mass-tagging also alleviates the need for intensive sample-preparation procedures and decreases the necessary resolving power of the mass spectrometer. The primers are extended, purified and then analyzed by MALDI-TOF mass spectrometry to determine the identity of the nucleotide present at the SNP position. MassARRAY.TM. (Sequenom, San Diego, Calif.) is a commercially available system for SNP identification using mass spectrometry.

The primer extension assay has also been modified to use fluorescence polarization as the means of detecting the specific nucleotide at the SNP position. This modified assay is sometimes referred to as template-directed dye-terminator incorporation assay with fluorescence polarization (FP-TDI). See Kwok (2002, supra). A kit for this assay, Acycloprimer.TM.-FP, is commercially available from Perkin Elmer (Boston, Mass.).

Allele-specific oligonucleotide ligation, also called oligonucleotide ligation assay (OLA) and is similar in many respects to ligase chain reaction, uses a pair of oligonucleotide probes that hybridize to adjacent segments of sequence on a nucleic acid fragment containing the SNP. One of the probes has a SNP allele-specific base at its 3' or 5' end. The second probe hybridizes to sequence that is common to all SNP alleles. If the first probe has an allele-specific base at its 3' end, the second probe hybridizes to the sequence segment immediately 3' to the SNP. If the first probe has an allele-specific base at its 5' end, the second probe hybridizes to the sequence segment immediately 5' to the SNP. The two probes can be ligated together only when both are hybridized to a DNA fragment containing the SNP allele for which the first probe is specific. See Landegren et al. (1988, Science 241:1077-80). One method of detecting the ligation product involves fluorescence. The second probe, which hybridizes to either allele, is fluorescently labeled. The allele-specific probe is labeled with biotin. Strepavidin capture of the allele-specific ligation product and subsequent fluorescent detection is used to determine which SNP is present. Another variation of this assay combines amplification and ligation in the same step (Barany, 1991, PNAS 88:189-93). A commercially available kit, SNPlex.TM. (Applied Biosystems, Foster City, Calif.) uses capillary electrophoresis to analyze the ligation products.

Allele-specific hybridization, also called allele-specific oligonucleotide hybridization (ASO), distinguishes between two DNA molecules differing by one base using hybridization. Amplified DNA fragments containing the target SNP are hybridized to allele-specific oligonucleotides. In one variation, the amplified DNA fragments are fluorescence labeled and the allele-specific oligonucleotides are immobilized. See, for instance, Strachan et al., (1999, In: Human Molecular Genetics, Second Edition, John Wiley & Sons, New York, N.Y.). In another variation, the allele-specific oligonucleotides are labeled with a antigen moiety. Binding is detected via an enzyme-linked immunoassay and color reaction (see, for instance, Knight et al., 1999, Clin. Chem. 45: 1860-1863). In yet another variation, the allele-specific oligonucleotides are radioactively labeled (see, for instance, Saiki et al., 1986, Nature 324:163-6). Protein nucleic acid (PNA) probes and mass spec may also be used (Ross et al., 1997, Anal. Chem. 69:4197-4202).

Allele-specific hybridization may also be performed by using an array of oligonucleotides, where discrete positions on the array are complementary to one or more of the provided polymorphic sequences, e.g. oligonucleotides of at least 12 nt, frequently 20 nt, or larger, and including the sequence flanking the polymorphic position. Such an array may comprise a series of oligonucleotides, each of which can specifically hybridize to a different polymorphism. For examples of arrays, see Hacia et al. (1996) Nature Genetics 14:441-447; Lockhart et al. (1996) Nature Biotechnol. 14:1675-1680; and De Risi et al. (1996) Nature Genetics 14:457-460.

Other SNP identification methods based on the formation of allele-specific complexes include the invasive cleavage assay and the branch migration assay. The invasive cleavage assay uses two probes that have a one nucleotide overlap. When annealed to target DNA containing the SNP, the one nucleotide overlap forms a structure that is recognized by a 5' nuclease that cleaves the downstream probe at the overlap nucleotide. The cleavage signal can be detected by various techniques, including fluorescence resonance energy transfer (FRET) or fluorescence polarization. Reaction conditions can be adjusted to amplify the cleavage signal, allowing the use of very small quantities of target DNA. Thus, the assay does not require amplication of the target prior to detecting the SNP identity, although an amplified sequence may be used. See, for instance, Lyamichev et al., 2003, Methods Mol. Biol 212:229-240; Brookes, 1999, Gene, 234:177-186; and Mein et al., 2000, Genome Res. 10:330-343). A commercially available product, the Invader.RTM. assay (Third Wave Molecular Diagnostics, Madison, Wis.), is based on this concept. The branch migration assay based on Holliday junction migration, involves the detection of a stable four-way complex for SNP identification (See, for instance, U.S. Pat. No. 6,878,530).

SNPs can also be scored by direct DNA sequencing. A variety of automated sequencing procedures may be utilized when performing the diagnostic assays (Naeve et al., 1995, Biotechniques 19:448-453), including sequencing by mass spectrometry (see, e.g., PCT International Publication No. WO 94/16101; Cohen et al., 1996, Adv. Chromatogr. 36:127-162; and Griffin et al., 1993, Appl. Biochem. Biotechnol. 38:147-159). Traditional sequencing methods may also be used, such as dideoxy-mediated chain termination method (Sanger et al., 1975, J. Molec. Biol. 94: 441; Prober et al. 1987, Science 238: 336-340) and the chemical degradation method (Maxam et al., 1977, PNAS 74: 560).

A preferred sequencing method for SNPs is pyrosequencing. See, for instance, Ahmadian et al., 2000, Anal. Biochem, 280:103-110; Alderbom et al., 2000, Genome Res. 10:1249-1258 and Fakhrai-Rad et al., 2002, Hum. Mutat. 19:479-485. Pyrosequencing involves a cascade of four enzymatic reactions that permit the indirect luciferase-based detection of the pyrophosphate released when DNA polymerase incorporates a dNTP into a template-directed growing oligonucleotide. Each dNTP is added individually and sequentially to the same reaction mixture, and subjected to the four enzymatic reactions. Light is emitted only when a dNTP is incorporated, thus signaling which dNTP in incorporated. Unincorporated dNTPs are degraded by apyrase prior to the addition of the next dNTP. The method can detect heterozygous individuals in addition to heterozygotes. Pyrosequencing uses single stranded template, typically generated by PCR amplification of the target sequence. One of the two amplification primers is biotinylated thereby enabling streptavidin capture of the amplified duplex target. Streptavidin-coated beads are useful for this step. The captured duplex is denatured by alkaline treatment, thereby releasing the non-biotinylated strand. The detection primer used for SNP identification using pyrosequencing is designed to hybridize to a sequence 3' to the SNP. In a preferred embodiment, the 3' sequence is immediately adjacent to the SNP position. Thus, the SNP identity is ascertained when the first nucleotide is incorporated. Pyrosequencing can detect heterozygotes.

Further examples of methods that can be used to identify for the SNPs of the present invention include single-strand conformational polymorphism (SSCP) and denaturing gradient gel electrophoresis (DGGE). SSCP identifies base differences by alteration in electrophoretic migration of single stranded PCR products, as described in Orita et al., (1989, PNAS 86:2766-1770). Single-stranded PCR products can be generated by heating or otherwise denaturing double-stranded PCR products. Single-stranded nucleic acids may refold or form secondary structures that are partially dependent on the base sequence. The different electrophoretic mobilities of single-stranded amplification products. are related to base-sequence differences at SNP positions. DGGE differentiates SNP alleles based on the different sequence-dependent stabilities and melting properties inherent in polymorphic DNA and the corresponding differences in electrophoretic migration patterns in a denaturing gradient gel (Myers et al., 1985, Nature 313:495 and Erlich, ed., 1992, In: PCR Technology, Principles and Applications for DNA Amplification, W. H. Freeman and Co, New York, Chapter 7).

Sequence-specific ribozymes (U.S. Pat. No. 5,498,531) can be used to score SNPs based on the development or loss of a ribozyme cleavage site. Perfectly matched sequences can be distinguished from mismatched sequences by nuclease cleavage digestion assays or by differences in melting temperature. If the SNP affects a restriction enzyme cleavage site, the SNP can be identified by alterations in restriction enzyme digestion patterns, and the corresponding changes in nucleic acid fragment lengths determined by gel electrophoresis. Immunoassay methods using antibodies specific for SNP alleles can be used for SNP detection. Southern and Northern blot analysis can also be utilized for nucleic acid analysis. See, for instance, Sambrook et al. (2001, Molecular Cloning: A Laboratory Manual, Cold Spring Harbor Laboratory Press, Cold Spring Harbor, N.Y.), Ausubel et al. (eds., 1997, Current Protocols in Molecular Biology, John Wiley & Sons, New York), and Gerhardt et al. (eds., 1994, Methods for General and Molecular Bacteriology, American Society for Microbiology, Washington, D.C.).

The invention encompasses diagnostic screening using SNPs that are genetically linked to a phenotypic variant in activity or expression. The SNP polymorphism itself need not be phenotypically expressed, but may be linked to sequences that result in altered activity or expression. Two polymorphic variants may be in linkage disequilibrium, i.e. where alleles show non-random associations between genes even though individual loci are in Hardy-Weinberg equilibrium. Linkage analysis may be performed alone, or in combination with direct detection of phenotypically evident polymorphisms. The use of SNPs for genotyping is illustrated in Golevleva et al. (1996) Am. J. Hum. Genet. 59:570-578; and in Underhill et al. (1996) P.N.A.S. 93:196-200.

One might wish to use a a method of determining rs2108622 genotype that does not directly measure DNA or RNA. For example, suitable methods would include assays that measure enzymatic activity or protein levels dependent upon CYP4F2.

### Kits related to SNPs of the Invention

The invention also provides a kit useful in practicing the method of the invention. The kit may contain at least one pair of amplication primers that is used to amplify a target polynucleotide sequence containing one of the SNPs identified in the invention. The amplification primers are designed based on the sequences provided herein for the upstream and downstream sequence flanking the SNPs. In a preferred embodiment, the amplification primers will generate an amplified double-stranded target polynucleotide between about 50 base pairs to about 600 base pairs in length and, more preferably, between about 100 base pairs to about 300 base pairs in length. In another preferred embodiment, the SNP is located approximately in the middle of the amplified double-stranded target polynucleotide.

The kit may further contain a detection probe designed to hybridize to a sequence 3' to the SNP on either strand of the amplified double-stranded target polynucleotide. In one variation, the detection probe hybridizes to the sequence immediately 3' to the SNP on either strand of the amplified double-stranded target polynucleotide but does not include the SNP. This kit variation may be used to identify the SNP by pyrosequencing or a primer extension assay. For use in pyrosequencing, one of the amplification primers in the kit may be biotinylated and the detection probe is designed to hybridize to the biotinylated strand of the amplified double-stranded target polynucleotide. For use in a primer extension assay, the kit may optionally also contain fluorescently labeled ddNTPs. Typically, each ddNTP has a unique fluorescent label so they are readily distinguished from each other.

Alternatively, the kit is designed for allele specific oligonucleotide ligation. In this embodiment, in addition to the at least one pair of amplification primers, the kit may further contain a pair of detection probes that hybridize to immediately adjacent segments of sequence in one of the strands of the target polynucleotide containing the SNP. One of the two probes is SNP-allele specific; it has a SNP allele-specific nucleotide at either its 5' or 3' end. The second probe hybridizes immediately adjacent to the first probe, but is not allele specific. In one variation, the allele-specific probe is fluorescently labeled and the second probe is biotinylated, such that if the two probes are ligated, the resultant ligation product is both fluorescently labeled and biotinylated. Optionally, a third probe may be provided which is specific for the other allele of the SNP. If the optional third probe is provided, its fluorescent label may be distinguishably different from the label on the first probe.

In yet another variation, the kit is designed for a 5' nuclease assay. In this variation, in addition to the at least one pair of amplification primers, the kit may further contain at least one SNP allele-specific probe which is fluorescently labeled. The allele-specific probe may hybridize to either strand of the amplified double-stranded target polynucleotide. In a preferred embodiment, the allele-specific probe evenly straddles the SNP. That is, the SNP position is approximately in the middle of the allele-specific probe. Optionally, the kit also contains a second allele-specific probe which is specific for another allele of the SNP for which the first probe is specific. The fluorescent label on the optional second probe may be distinguishably different from the label on the first probe.

Any of the above kit variations may contain sets of primers and probes for more than one SNP position. For instance, the SNPs detected may be any combination of the SNPs taught herein, including all of the SNPs. Probes and/or primers for other SNPs diagnostic for a particular disease or malady may also be included. Any kit may optionally contain one or more nucleic acids that serve as a positive control for the amplification primers and/or the probes. Any kit may optionally contain an instruction material for performing risk diagnosis.

The following examples are, of course, offered for illustrative purposes only, and are not intended to limit the scope of the present invention in any way. Indeed, various modifications of the invention in addition to those shown and described herein will become apparent to those skilled in the art from the foregoing description and the following examples and fall within the scope of the appended claims.

### EXAMPLES

### Background

Warfarin is an effective, commonly-prescribed anticoagulant used to treat and prevent thrombotic events. Despite its low cost and lack of alternative drugs, warfarin remains under-prescribed because of historically high rates of drug-associated adverse events. Further, inter-individual variability in therapeutic dose mandates frequent monitoring until target anticoagulation is achieved. Genetic polymorphisms occurring in enzymes involved in metabolism of warfarin have been implicated in variability of dose. This study describes a novel variant that influences warfarin dose requirements.

### Methods

To identify additional genetic variants that contribute to warfarin requirements, screening of DNA variants in additional genes that code for drug metabolizing enzymes and drug transport proteins was undertaken using the Affymetrix drug-metabolizing enzymes and transporters panel.

### Results

A DNA variant (rs2108622; V433M) in cytochrome P450 4F2 (CYP4F2) was associated with warfarin dose in three independent Caucasian cohorts of patients stabilized on warfarin representing diverse geographic regions in the USA and accounted for a difference in warfarin dose of about 1 mg/day.

### Conclusion

Genetic variation of CYP4F2 was associated with a clinically important impact on warfarin dose requirement.

Warfarin is the only oral anticoagulant approved by the United States Food and Drug Administration. Although it has been used for over 50 years, initiation of therapy remains problematic due to inter-individual variability in degree of anticoagulation achieved in response to the same warfarin dose. Thus, an appropriate warfarin dose in one patient may induce a hemorrhagic event in another patient.

Warfarin initiation is associated with the second highest adverse event rate for a single drug.¹⁻⁴ Because of concerns regarding warfarin-induced bleeding, particularly in the elderly, physicians are often reluctant to initiate warfarin therapy in patients where it is warranted. Up to one-half of patients with atrial fibrillation and no contraindication to warfarin therapy, who are also at high risk of stroke (annual risk >4%), are currently not receiving anticoagulation therapy due to the risk perceived to be associated with such therapy by both patients and health care providers.⁵ Thus, more reliable dosing strategies for warfarin initiation that approximate optimal maintenance dosing could improve the risk-benefit ratio, allowing a broader range of patients to safely benefit from warfarin treatment.

Modeling stable dose requirements based on clinical, physiological, environmental, and genetic factors has shown promise as a strategic approach to predict individualized stable warfarin dose requirements.⁶⁻¹³ Patients who have variant alleles of cytochrome P450 (CYP) 2C9, the primary enzyme that metabolizes S-warfarin, require reduced maintenance doses compared to those having wild-type alleles.¹⁴⁻¹⁶ Warfarin dosing variability is also attributable to genetic polymorphisms in vitamin K 2,3 epoxide reductase complex 1 (VKORC1),^{6,17-20} the rate-limiting enzyme in the warfarin sensitive, vitamin K-dependent gamma carboxylation system. By incorporating these factors, it may be possible to decrease time to achieve stable dose and risk of serious or life-threatening hemorrhagic events in patients with variant alleles compared to patients with the wild-type genotype. To date, however, most pharmacogenetic models explain just over one-half of the variation in warfarin dose,⁶⁻¹⁰ suggesting that additional genetic, environmental, medical, or personal factors are important.

Genetic screening utilizing the Affymetrix drug-metabolizing enzymes and transporters (DMET) chip was undertaken to explore whether additional polymorphisms of drug metabolizing enzymes might contribute to warfarin dosing variability in a clinically important way. Here we provide evidence that a polymorphism in CYP4F2 further affects warfarin dose requirements. and speculate on its mechanism of action in the vitamin K pathway.

### MATERIALS AND METHODS

### Participating Subjects

### Screening/Discovery Cohort

The initial cohort was recruited at Marshfield Clinic, a multi-specialty group practice in Wisconsin as described.^{6,16} Patients were excluded from the study if they were known to have cancer, renal or hepatic insufficiency, or congestive heart failure.

### Validation Cohorts

A second cohort of patients stabilized on warfarin therapy was recruited at the University of Florida as described.⁷ Patients were excluded from this study if they had liver cirrhosis, advanced malignancy, hospitalization within the previous 4 weeks of the index visit, or febrile/diarrheal illness within 2 weeks of the index visit.

A third cohort of patients was recruited at Washington University in St. Louis as described.²¹ Patients were excluded from this study if they had liver cirrhosis, advanced malignancy, or febrile/diarrheal illness within 2 weeks of the index visit.

Protocols at all three institutions were approved by the respective Institutional Review Board, and all subjects provided informed written consent for participation, and use of their genetic sample in studies aimed at understanding warfarin dose variability.

### DMET Panel Testing

The targeted human DMET 1.0 assay (Affymetrix, Inc, Santa Clara, CA) utilizes molecular inversion probes and GeneChip universal tag arrays to target 1228 single nucleotide polymorphisms (SNP) within 170 genes for metabolic enzymes and transport and regulatory proteins (Table 2). The specificity of genotyping is provided by a gap in the molecular inversion probe that can only be filled with the nucleotide that is complementary to the target locus followed by ligation and amplification.^{22,23} The DMET assay has demonstrated high accuracy rates with 99.8% correctly called genotypes.²⁴

| **Table 2. Genes Containing SNPs with >1% Minor Allele Frequency in the Marshfield Population.** | | | | | | | |
|---|---|---|---|---|---|---|---|
| | **DMET Metabolism** | | | | **DMET Transport and Other Genes** | | |
| **Gene** | **SNP Count** | **Gene** | **SNP Count** | **Gene** | **SNP Count** | **Gene** | **SNP Count** |
| ADH1B | 3 | CYP2C18 | 4 | GSTM4 | 2 | ABCB1 | 14 |
| ADH4 | 4 | CYP2C19 | 1 | GSTO1 | 1 | ABCB4 | 9 |
| ADH7 | 2 | CYP2D6 | 7 | GSTP1 | 3 | ABCB11 | 15 |
| ALDH1A1 | 1 | CYP2E1 | 7 | HNMT | 1 | ABCC1 | 10 |
| ALDH2 | 1 | CYP2F1 | 2 | MAOB | 1 | ABCC2 | 8 |
| ALDH3A1 | 2 | CYP2J2 | 1 | NAT1 | 7 | ABCC3 | 3 |
| AOX1 | 1 | CYP2S1 | 1 | NAT2 | 6 | ABCC4 | 12 |
| CDA | 2 | CYP39A1 | 2 | NQO1 | 4 | ABCC5 | 3 |
| CHST1 | 1 | CYP3A4 | 2 | POR | 1 | ABCC6 | 4 |
| CHST2 | 1 | CYP3A43 | 3 | PTGIS | 1 | ABCG2 | 1 |
| CHST3 | 13 | CYP3A5 | 1 | SULT1A1 | 1 | AHR | 2 |
| CHST5 | 4 | CYP3A7 | 1 | SULT1A2 | 1 | ATP7A | 1 |
| CHST7 | 1 | CYP4A11 | 1 | SULT1A3 | 1 | ATP7B | 10 |
| CHST10 | 5 | CYP4B1 | 6 | SULT1C1 | 1 | NR3C1 | 3 |
| CHST11 | 7 | CYP4F2 | 7 | SULT1C2 | 1 | PPARD | 38 |
| CHST13 | 2 | CYP4F8 | 4 | SULT1E1 | 2 | PPARG | 1 |
| CHST8 | 1 | CYP4F11 | 5 | SULT2A1 | 1 | RALBP1 | 3 |
| COMT | 2 | CYP4F12 | 4 | SULT2B1 | 2 | SLC10A1 | 1 |
| CYP11A1 | 1 | CYP4Z1 | 1 | SULT4A1 | 2 | SLC10A2 | 7 |
| CYP11B1 | 10 | CYP51A1 | 3 | TBXAS1 | 2 | SLC13A1 | 2 |
| CYP11B2 | 4 | CYP7A1 | 1 | TPMT | 3 | SLC15A1 | 5 |
| CYP17A1 | 3 | CYP8B1 | 1 | UGT1A1 | 5 | SLC15A2 | 5 |
| CYP19A1 | 5 | DPYD | 2 | UGT1A3 | 3 | SLC16A1 | 2 |
| CYP1A1 | 2 | EPHX1 | 7 | UGT1A4 | 1 | SLC19A1 | 2 |
| CYP1A2 | 6 | FMO1 | 4 | UGT1A6 | 2 | SLC22A1 | 7 |
| CYP1B1 | 1 | FMO2 | 10 | UGT1A7 | 1 | SLC22A2 | 2 |
| CYP20A1 | 2 | FMO3 | 6 | UGT2A1 | 2 | SLC22A3 | 2 |
| CYP24A1 | 4 | FMO5 | 1 | UGT2B4 | 2 | SLC22A4 | 3 |
| CYP2A13 | 3 | FMO6 | 4 | UGT2B11 | 1 | SLC22A5 | 3 |
| CYP2A6 | 13 | GSTA1 | 1 | UGT2B15 | 1 | SLC22A6 | 1 |
| CYP2A7 | 3 | GSTA2 | 4 | UGT2B28 | 1 | SLC22A8 | 2 |
| CYP2B6 | 8 | GSTA4 | 4 | UGT8 | 1 | SLC28A1 | 8 |
| CYP2C8 | 2 | GSTA5 | 1 | XDH | 4 | SLC28A2 | 1 |
| CYP2C9 | 2 | GSTM3 | 2 | | | SLC28A3 | 2 |
| | | | | | | SLC29A1 | 1 |
| | | | | | | SLC5A6 | 2 |
| | | | | | | SLC7A5 | 1 |
| | | | | | | SLC7A7 | 5 |
| | | | | | | SLCO1A2 | 3 |
| | | | | | | SLCO1B1 | 5 |
| | | | | | | SLCO1B3 | 4 |
| | | | | | | SLCO2B1 | 1 |
| | | | | | | SPG7 | 2 |

| | | | | | | | |
|---|---|---|---|---|---|---|---|
| DMET, drug-metabolizing enzymes and transporters; SNP, single nucleotide polymorphism | | | | | | | |

The laboratory protocol followed the standard process described in the Targeted Genotyping System User Guide²⁵ except for a preliminary polymerase chain reaction (PCR)-amplification step aimed at resolving genotypes from 29 SNPs in regions containing pseudogenes and close homologues. GeneChip Targeted Genotyping Analysis software was used to make genotype calls and generate QC metrics including call rates, average signal, cluster quality, Hardy-Weinberg equilibrium, and reproducibility. Of the 1228 SNP assays, 11 failed due to low call rate and were removed from the analysis. Genotypes represented by the remaining 1217 SNP assays were called according to either predefined boundaries (derived from historical training sets) or dynamic clustering using the current data set. From an initial sample population of 497 patients, 491 passed all QC metrics and produced useable genotypes. In the nearly homogenous Caucasian Marshfield population, 517 SNPs across 144 genes showed a minor allele frequency >1% and were utilized in the association study. The remaining polymorphisms target rare mutations or SNPs prevalent in other racial groups.

### Genotyping Methods

VKORC1 genotypes were determined on 436 Marshfield Clinic samples as described⁶ and on the remaining 61 samples using Invader® chemistry in a laboratory test developed with analyte specific reagents manufactured by Third Wave Technologies (TWT, Madison, WI). The laboratory developed assay used analyte-specific reagents that detect CYP2C9*2, CYP2C9*3, and VKORC1 (position -1639).²⁶

### Genotyping Quality Control Procedures

To confirm results from the DMET panel, CYP4F2 genotypes were subsequently validated with 100% concordance for 9 samples using Big Dye Terminator v3.1 cycle sequencing with results read on an Applied Biosystems Prism™ 3100 Genetic Analyzer. Twenty-one additional samples were tested using Invader^{®} CYP4F2 RUO reagents provided by TWT with 100% concordance of genotype. This TWT assay was previously used in the Japanese Millennium project^{27,28} and the International Haplotype Map project. Analytical performance of the TWT RUO assay was validated using synthetic targets for both alleles and four sets of trios from the HapMap project obtained from Coriell Institute for Medical Research (Camden, NJ).

Quality control procedures for CYP4F2 at the University of Florida and Washington University in St. Louis were blind duplicate genotyping. Genotyping of 15 samples of known genotype was also performed at the University of Florida.

### Statistical Analysis

Candidate SNPs were screened for association with the residuals from the Marshfield pharmacogenetic model⁶ for 429 Marshfield subjects. The Kruskal-Wallis test was used to compare the distribution of residuals among observed SNP categories for each SNP. Because a large number of SNPs were to be screened, a Bonferroni correction to the usual 5% nominal level (P<0.05/1228=0.00004) was set as a stringent initial threshold for statistical significance.

To validate the single SNP (rs2108622) that stood out strongly and was biologically plausible, the result was initially validated in 61 additional Marshfield subjects. The observed association was next validated in two independent cohorts from the University of Florida (292 Caucasian subjects) and Washington University in St. Louis (269 Caucasian subjects). These data were classified according to SNP genotype and evaluated relative to raw therapeutic dose and residuals from the Marshfield model.

In a final set of analyses, data from all three sites were pooled (n=1051 total subjects) and used to fit a series of new regression models in order to fully evaluate the potential contribution of rs2108622 to inter-individual variability in therapeutic warfarin dose, and to investigate the consistency of the effect across sites.

All models were fit with the log of stable warfarin dose as the response. The association of CYP2C9 and therapeutic warfarin dose has been well established with substantially reduced stable dose requirements in individuals with no wild type CYP2C9 alleles (i.e., no * 1 alleles). However, given the magnitude of effect and relatively low frequency of those alleles, even this large study did not include a sufficient number of subjects with these genotypes to reliably estimate the independent impact of clinical and genetic predictors. Therefore, we conducted multiple regression analyses only on the * 1 genotypes (n=1009), and used the observed mean dose (log scale) as the predicted value for those with only non-wild CYP2C9 alleles (*2/*2, *2/*3, and *3/*3, n= 42). Accordingly, our models that include genetic variables are composites of multiple regression and simple mean-based estimation approaches.

To assess the relative contribution of clinical and genetic factors, three models were developed using different sets of potential predictor variables: clinical factors only (gender, age, body surface area, and target international normalized ratio), clinical factors plus CYP2C9 and VKORC1, and clinical factors CYP2C9, VKORC1, and rs2108622. A modified step-down approach²⁹ was used, starting with a "full" model which included all potential predictors pertinent to that model, effects to allow the three study sites to differ, two-factor interactions (including those by site), and quadratic terms for age, body surface area, and target international normalized ratio. Terms were then cautiously excluded from this full model if they showed very weak associations with dose (P<0.5 in the initial steps, P<0.1 for the final model). The regression models were finalized after evaluating residuals and excluding cases with large residuals as outliers (|Studentized Residual| >3). At most 10 cases (<1% of the cohort) were excluded as outliers during model fitting (these outliers were included for model assessment, as described below).

The explanatory power for the three final models was assessed graphically and by using the adjusted r-squared (R²_{adj}). The R²_{adj} statistic measures the proportion of the total variability explained by the model with adjustment for the number of parameters in the model. The statistic was directly calculated to use all cases under analysis by pooling across both parts of the composite models and by applying the final regression model to any outliers dropped when estimating the parameters in the regression models.

### RESULTS

### Discovery of Novel SNPs Affecting Warfarin Dose Requirements

We used the DMET genotyping platform to assay 1228 SNPs. Of these, 517 were >1% polymorphic in the Marshfield Caucasian population. The 517 polymorphisms are distributed within 144 genes thought to be medically relevant (Table 2).

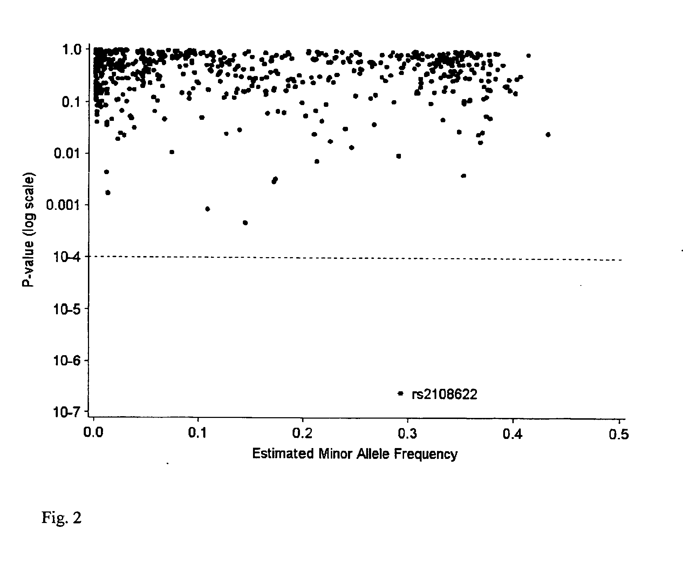

In our initial screening analysis, we tested each SNP for association with the residual variability from the Marshfield prediction model, evaluating each SNP for the proportion of residual variability explained by it after adjusting for known clinical factors and CYP2C9 and VKORC1. One SNP, rs2108622, which represents a polymorphism in CYP4F2, stood out dramatically in the analysis (Figure 2). The estimated P-value of 2.4 x 10⁻⁷ was well below our stringent initial threshold for statistical significance of 4.0 x 10⁻⁵.

Three additional SNPs, rs3093114, rs3093106 and rs3093105, were in partial linkage disequilibrium with rs2108622. Each showed strong association with warfarin therapeutic dose, although none of these SNPs attained statistical significance under our stringent threshold.

### Validation of SNP rs2108622

Results for 61 new Marshfield cases were obtained for initial confirmation of the association between rs2108622 and warfarin dose. In these new cases, the residuals showed association with rs2108622 (P=0.023). Based on validation in this small Marshfield sample, the association was further tested in two independent cohorts from geographically distinct regions of the country.

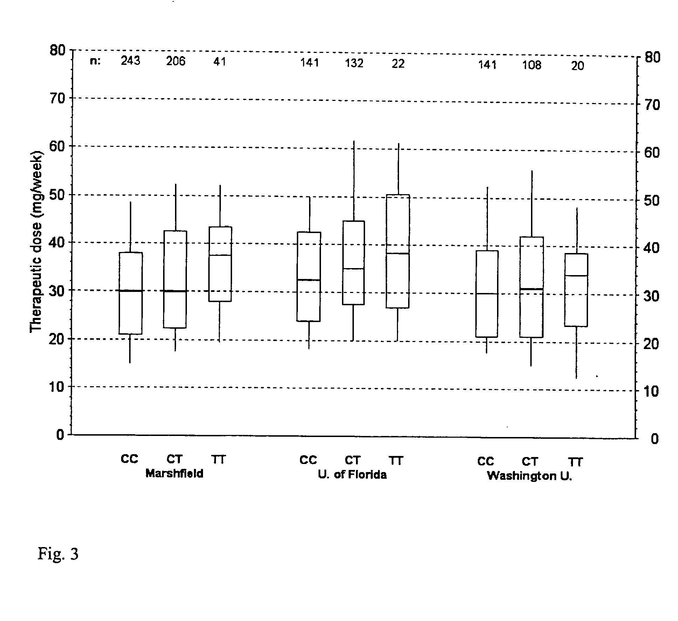

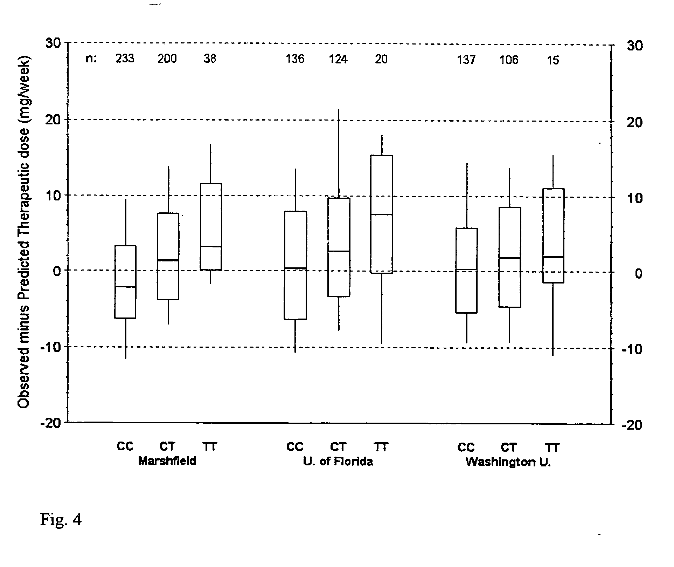

Figure 3 depicts the distribution of warfarin therapeutic dose by CYP4F2 genotype for the Marshfield cohort and the two validation cohorts. The raw data in Figure 3 have not been adjusted for other clinical or genetic factors, but by inspection of the relationships between dose and CYP4F2, genetic variants are consistent across the three study groups with CC homozygotes requiring less warfarin. TT homozygotes required more warfarin than predicted and heterozygotes (CT) required intermediate warfarin doses. This pattern is repeated in Figure 4 where we have controlled for clinical factors, as well as CYP2C9 and VKORC1 genotypes, by evaluating the distributions of residuals from the Marshfield model. Site-specific analyses of these relationships yielded statistically significant results for the Marshfield (P<0.001) and University of Florida (P=0.027) study cohorts; results for the Washington University in St. Louis cohort (P=0.382) were not statistically significant but the trend was consistent across cohorts.

Multiple regression models were fit to the combined data from all three sites in order to better assess the overall contribution of CYP4F2 to variability in dose. Results from the models are summarized by predictor contribution to variation in therapeutic warfarin dose in Table 3. Clinical factors alone explained about 16% of the overall variability among patients in therapeutic warfarin dose. Clinical factors together with CYP2C9 and VKORC1 genotype explained about 53% of the variability, while the addition of CYP4F2 genotype increased this R²_{adj} to 55%.

Site-specific effects were evaluated in the multiple regression models in order to examine the consistency of dosing and CYP4F2 effects. Some difference in the general level of dosing was observed by site (Figure 3), with somewhat higher doses at the University of Florida (P=0.019) and somewhat lower doses at Washington University in St. Louis (P=0.056) relative to Marshfield. With respect to CYP4F2, site effects were significant at Washington University in St. Louis (P=0.018) but not the University of Florida (P=0.105) relative to Marshfield. Site-specific estimates of the CYP4F2 effect in the final pooled model showed a 12.8% increase with each T allele at Marshfield, as compared with 6.8% at the University of Florida and 3.8% at Washington University. in St. Louis.

### DISCUSSION

We have identified a DNA variant in CYP4F2 that has a clinically important impact on stable warfarin dose. In our pooled analyses, patients having two TT alleles require about 1 mg/day more warfarin than patients having two CC alleles.

Because it is frequently difficult to generalize the original association between a gene variant and phenotype,³⁰ we replicated our original observation in two additional Caucasian cohorts of patients stabilized on warfarin. Importantly, all three cohorts demonstrated a consistent effect of CYP4F2 rs2108622 on warfarin dose. However, as expected, the absolute contribution of CYP4F2 to stable warfarin dose in each cohort varied from a high of 26% for Marshfield to a low of 8% for Washington University in St. Louis.

We postulate that the observed site-related variation in effect may be due to differences among cohorts with respect to other genetic and clinical factors not included in our current model. However, since no other studies have pooled data from multiple, independent sites to estimate the effects of polymorphisms on warfarin stable dose, we do not know whether the effects we have observed are typical of pooled site models as compared to single site models. This would more likely be the result if single site models were developed on populations that were more genetically homogeneous as is the case with the Marshfield model.

Because of differences in the frequency of the underlying genetic variants among major racial groups, the potential clinical benefit from prospective CYP4F2 genotyping varies by race. In Caucasians and Asians, the minor allele frequency for CYP4F2 is ~30% compared to ~7% in African-Americans. Accordingly, from a population perspective, the expected contribution of this polymorphism to stable warfarin dose in African-Americans is likely to be less than in Caucasians and Asians.

The physiological role of CYP4F2 in the vitamin K/warfarin pathway is unknown. It is known, however, that CYP4F2 hydroxylates the tocopherol phytyl side chain as the first step in the inactivation pathway of vitamin E.³¹ Given the similarity of the vitamin E and vitamin K side chain, CYP4F2 may hydroxylate the vitamin K phytyl side chain. In contrast, it has also been demonstrated that CYP4F2 is the major cytochrome responsible for synthesis of 20-hydroxyeicosatetraenoic acid in human kidney.³² Whether the effect of CYP4F2 on the vitamin K/warfarin pathway is mediated through 20-HETE production remains to be tested.

It is known that the polymorphism in CYP4F2 affects enzyme activity. This polymorphism decreased 20-HETE production in a reconstituted recombinant protein system to about 60% of the wild-type enzyme.³² We postulate that individuals with T alleles have decreased function of the enzyme thereby increasing the individual's requirement for warfarin.

Although recent studies^{12,13,32} demonstrate that using dosing models that include genetic testing may improve warfarin dosing, we have not presented a clinical model for use in prospectively dosing patients initiating warfarin therapy. However, we expect that such models will be forthcoming and that these models will continue to evolve as data from more patients at additional sites become available for evaluation. We also expect that as those models improve with the discovery of additional genes or other factors, they will be important in clinical warfarin dosing.

| Table 3**. Explained Variation in Therapeutic Warfarin Dose Pooled Models.** | |
|---|---|
| **Predictor/Predictor Group** | **Adjusted R²** |
| Clinical only | 0.160 |
| Clinical plus CYP2C9 and VKORC1 | 0.529 |
| Clinical plus CYP2C9 and VKORC1 and CYP4F2 | 0.548 |

| | |
|---|---|
| Clinical variables were: gender, age, body surface area, and target international normalized ratio. | |

### References

1. Petty GW, Brown RD Jr, Whisnant JP, Sicks JD, O'Fallon WM, Wiebers DO. Frequency of major complications of aspirin, warfarin, and intravenous heparin for secondary stroke prevention. A population-based study. Ann Intern Med 1999;130:14-22.
2. van der Meer FJ, Rosendaal FR, Vandenbroucke JP, Briet E. Bleeding complications in oral anticoagulant therapy. An analysis of risk factors. Arch Intern Med 1993;153:1557-62.
3. Cannegieter SC, Rosendaal FR, Wintzen AR, van der Meer FJ, Vandenbroucke JP, Briet E. Optimal oral anticoagulant therapy in patients with mechanical heart valves. N Engl J Med 1995;333:11-7.
4. Palareti G, Leali N, Coccheri S, et al. Bleeding complications of oral anticoagulant treatment: an inception-cohort, prospective collaborative study (ISCOAT). Italian Study on Complications of Oral Anticoagulant Therapy. Lancet 1996;348:423-8.
5. Evans A, Davis S, Kilpatrick C, Gerraty R, Campbell D, Greenberg P. The morbidity related to atrial fibrillation at a tertiary centre in one year: 9.0% of all strokes are potentially preventable. J Clin Neurosci 2002;9:268-72.
6. Caldwell MD, Berg RL, Zhang KQ, et al. Evaluation of genetic factors for warfarin dose prediction. Clin Med Res 2007;5:8-16.
7. Aquilante CL, Langaee TY, Lopez LM, et al. Influence of coagulation factor, vitamin K epoxide reductase complex subunit 1, and cytochrome P450 2C9 gene polymorphisms on warfarin dose requirements. Clin Pharmacol Ther 2006;79:291-302.
8. Zhu Y, Shennan M, Reynolds KK, et al. Estimation of warfarin maintenance dose based on VKORC1 (-1639 G>A) and CYP2C9 genotypes. Clin Chem 2007;53:1199-205.
9. Gage BF. Pharmacogenetics-based coumarin therapy. Hematology Am Soc Hematol Educ Program 2006;:467-73.
10. Sconce EA, Kamali F. Appraisal of current vitamin K dosing algorithms for the reversal of over-anticoagulation with warfarin: the need for a more tailored dosing regimen. Eur J Haematol 2006;77:457-62.
11. Millican EA, Lenzini PA, Milligan PE, et al. Genetic-based dosing in orthopedic patients beginning warfarin therapy. Blood 2007;110:1511-5.
12. Hillman MA, Wilke RA, Yale SH, et al. A prospective, randomized pilot trial of model-based warfarin dose initiation using CYP2C9 genotype and clinical data. Clin Med Res 2005;3:137-45.
13. Caraco Y, Blotnick S, Muszkat M. CYP2C9 genotype-guided warfarin prescribing enhances the efficacy and safety of anticoagulation: a prospective randomized controlled study. Clin Pharmacol Ther 2007 Sept 12 [Epub ahead of print].
14. Aithal GP, Day CP, Kesteven PJ, Daly AK. Association of polymorphisms in the cytochrome P450 CYP2C9 with warfarin dose requirement and risk of bleeding complications. Lancet 1999;353:717-9.
15. Peyvandi F, Spreafico M, Siboni SM, Moia M, Mannucci PM. CYP2C9 genotypes and dose requirements during the induction phase of oral anticoagulant therapy. Clin Pharmacol Ther 2004;75:198-203.
16. Hillman MA, Wilke RA, Caldwell MD, Berg RL, Glurich I, Burmester JK. Relative impact of covariates in prescribing warfarin according to CYP2C9 genotype. Pharmacogenetics 2004;14:539-47.
17. Rieder MJ, Reiner AP, Gage BF, et al. Effect of VKORC1 haplotypes on transcriptional regulation and warfarin dose. N Engl J Med 2005;352:2285-93.
18. D'Andrea G, D'Ambrosio RL, Di Perna P, et al. A polymorphism in the VKORC1 gene is associated with an interindividual variability in the dose-anticoagulant effect of warfarin. Blood 2005;105:645-9.
19. Wadelius M, Chen LY, Downes K, et al. Common VKORC1 and GGCX polymorphisms associated with warfarin dose. Pharmacogenomics J 2005;5:262-70.
20. Crawford DC, Ritchie MD, Rieder MJ. Identifying the genotype behind the phenotype: a role model found in VKORC1 and its association with warfarin dosing. Pharmacogenomics 2007;8:487-96.
21. Gage BF, Eby C, Milligan PE, Banet GA, Duncan JR, McLeod HL. Use of pharmacogenetics and clinical factors to predict the maintenance dose of warfarin. Thromb Haemost 2004;91:87-94.
22. Hardenbol P, Baner J, Jain M, et al. Multiplexed genotyping with sequence-tagged molecular inversion probes. Nat Biotechnol 2003;21:673-8.
23. Hardenbol P, Yu F, Belmont J, et al. (2005). Highly multiplexed molecular inversion probe genotyping: over 10,000 targeted SNPs genotyped in a single tube assay. Genome Res 2005;15:269-75.
24. Daly TM, Dumaual CM, Miao X, et al. Multiplex assay for comprehensive genotyping of genes involved in drug metabolism, excretion, and transport. Clin Chem 2007;53:1222-30.
25. Affymetrix® GeneChip® Scanner 3000 targeted genotyping system user guide. (Accessed July 17, 2007, at www.affymetrix.com/support/downloads/manuals/scanner3000_targeted_genotyping_user_guide.pdf. )
26. Koelbl JA, Mielke C, Stodola AJ, et al. Genotyping of CYP2C9 and VKORC1 polymorphisms using Invader® chemistry. J Mol Diagn 2006;8:625.[abstract G14]
27. Haga H, Yamada R, Ohnishi Y, Nakamura Y, Tanaka T. Gene-based SNP discovery as part of the Japanese Millennium Genome Project: identification of 190,562 genetic variations in the human genome. Single-nucleotide polymorphism. J Hum Genet 2002;47:605-10.
28. Ohnishi Y, Tanaka T, Ozaki K, Yamada R, Suzuki H, Nakamura Y. A high-throughput SNP typing system for genome-wide association studies. J Hum Genet 2001;46:471-7.
29. Harrell FE, Lee KL, Mark DB. Multivariable prognostic models: issues in developing models, evaluating assumptions and adequacy, and measuring and reducing errors. Stat Med 1996;15:361-87.
30. NCI-NHGRI Working Group on Replication in Association Studies, Chanock SJ, Manolio T, et al. Replicating genotype-phenotype associations. Nature 2007;447:655-60.
31. Sontag TJ, Parker RS. Cytochrome P450 omega-hydroxylase pathway of tocopherol catabolism. Novel mechanism of regulation of vitamin E status. J Biol Chem 2002;277:25290-6.
32. Stec DE, Roman RJ, Flasch A, Rieder MJ. Functional polymorphism in human CYP4F2 decreases 20-HETE production. Physiol Genomics 2007;30:74-81.
33. Voora D, Eby C, Linder MW, et al. Prospective dosing of warfarin based on cytochrome P-450 2C9 genotype. Thromb Haemost 2005;93:700-5.

## Claims

1. A method for optimising an anticoagulant dosage for a patient requiring the anticoagulant, wherein the anticoagulant is a coumarin based anticoagulant, said method comprising:
determining the CYP4F2 genotype of the patient; and
determining the anticoagulant dosage for the patient as a function of variables, wherein at least one variable comprises the CYP4F2 genotype.

2. The method of claim 1, wherein the variables further comprise the patient's age, gender, body surface area, diabetic condition, presence of artificial heart valve, or a combination thereof.

3. The method of claim 1, wherein the variables further comprise the patient's CYP2C9 or VKORC1 genotype.

4. The method of claim 1, wherein said method optimises the initial anticoagulant dosage for the patient.

5. The method of claim 4 further comprising optimising a new anticoagulant dose regimen.

6. The method of claim 5, wherein the new anticoagulant dose regimen calculation is based on calculation factors comprising the patient's current anticoagulation medication dose and a new dosage factor comprising current international normalized ratio, international normalized ratio goal, or a combination thereof.

7. The method of any one of the preceding claims, wherein the coumarin based anticoagulant is warfarin, a warfarin sodium salt, a warfarin derivative or a coumarin derivative.

8. The method of claim 7, wherein the warfarin derivative is selected from 6-, 7-, 8-, 4'-hydroxy, 6-chloro- and 6-bromowarfarin, 3, 4-dihydro-2*H*-pyran ketals, acyl and aryl warfarin derivatives, and 4-Hydrosy-3-[1-(4-chlorophenl)-3-oxobutyl]-2*H*-1- benzopyran-2-one, oxime.

9. The method of claim 1 wherein the CYP4F2 genotype is deduced through analysis of rs2108622.

10. The method of claim 1 wherein the CYP4F2 genotype is deduced through analysis of a SNP selected from rs3093114, rs3093106 or rs3093105.

## Patentansprüche

1. Verfahren zur Optimierung einer Antikoagulans-Dosierung für einen Patienten, der das Antikoagulans benötigt, wobei das Antikoagulans ein Coumarin-basiertes Antikoagulans ist, wobei das Verfahren umfasst:
Bestimmen des CYP4F2-Genotyps des Patienten und
Bestimmen der Antikoagulans-Dosierung für den Patienten als eine Funktion von Variablen, wobei wenigstens eine Variable den CYP4F2-Genotyp umfasst.

2. Verfahren gemäß Anspruch 1, wobei die Variablen außerdem das Alter, das Geschlecht, die Körperoberfläche, den diabetischen Zustand des Patienten, das Vorliegen einer künstlichen Herzklappe bei dem Patienten oder eine Kombination davon umfassen.

3. Verfahren gemäß Anspruch 1, wobei die Variablen außerdem den CYP2C9- oder VKORCl-Genotyp des Patienten umfassen.

4. Verfahren gemäß Anspruch 1, wobei das Verfahren die anfängliche Antikoagulans-Dosierung für den Patienten optimiert.

5. Verfahren gemäß Anspruch 4, das außerdem Optimieren eines neuen Antikoagulans-Dosis-Plans umfasst.

6. Verfahren gemäß Anspruch 5, wobei die Berechnung des neuen Antikoagulans-Dosis-Plans auf Berechnungsfaktoren basiert, die die gegenwärtige Antikoagulationsmedikations-Dosis und einen neuen Dosierungsfaktor, der ein gängiges internationales normalisiertes Verhältnis, ein internationales normalisiertes Verhältnisziel oder eine Kombination davon umfasst, umfassen.

7. Verfahren gemäß einem der vorangehenden Ansprüche, wobei das Coumarin-basierte Antikoagulans Warfarin, ein Warfarin-Natriumsalz, ein Warfarin-Derivat oder ein Coumarin-Derivat ist.

8. Verfahren gemäß Anspruch 7, wobei das Warfarin-Derivat aus 6-, 7-, 8-, 4'-Hydroxy-, 6-Chlor- und 6-Bromwarfarin, 3,4-dihydro-2H-Pyranketalen, Acyl- und Aryl-Warfarin-Derivaten und 4-Hydroxy-3-[1-(4-chlorphenl)-3-Oxobutyl]-2H-1-benzopyran-2-on, Oxim ausgewählt ist.

9. Verfahren gemäß Anspruch 1, wobei der CYP4F2-Genotyp durch Analyse von rs2108622 abgeleitet wird.

10. Verfahren gemäß Anspruch 1, wobei der CYP4F2-Genotyp durch Analyse eines SNP, ausgewählt aus rs3093114, rs3093106 oder rs3093105, abgeleitet wird.

## Revendications

1. Procédé d'optimisation de la posologie d'un anticoagulant pour un patient qui a besoin de cet anticoagulant, dans lequel procédé l'anticoagulant est un anticoagulant à base de coumarine, et lequel procédé comporte les étapes suivantes :
- déterminer le génotype CYP4F2 du patient ;
- et déterminer la posologie de l'anticoagulant pour le patient en fonction de plusieurs variables, étant entendu qu'au moins une variable englobe le génotype CYP4F2.

2. Procédé conforme à la revendication 1, dans lequel les variables comprennent en outre l'âge du patient, son sexe, sa surface corporelle, son statut diabétique, la présence d'une valvule cardiaque artificielle, ou une combinaison de ces variables.

3. Procédé conforme à la revendication 1, dans lequel les variables englobent en outre le génotype CYP2C9 ou VKORC1 du patient.

4. Procédé conforme à la revendication 1, lequel procédé permet d'optimiser la posologie initiale de l'anticoagulant pour le patient.

5. Procédé conforme à la revendication 4, comprenant en outre l'optimisation d'un nouveau schéma posologique de l'anticoagulant.

6. Procédé conforme à la revendication 5, dans lequel le calcul du nouveau schéma posologique de l'anticoagulant est fondé sur des paramètres de calcul comprenant la dose actuelle de médicament anti-coagulant administrée au patient, et un facteur de nouvelle posologie comprenant un rapport normalisé international actuel, un rapport normalisé international recommandé, ou une combinaison de ceux-ci.

7. Procédé conforme à l'une des revendications précédentes, dans lequel l'anticoagulant à base de coumarine est la warfarine, le sel de sodium de la warfarine, un dérivé de la warfarine ou un dérivé de la coumarine.

8. Procédé conforme à la revendication 7, pour lequel le dérivé de la warfarine est choisi parmi les 6-hydroxy-warfarine, 7-hydroxy-warfarine, 8-hydroxy-warfarine, 4'-hydroxy-warfarine, 6-chloro-warfarine et 6-bromo-warfarine, les acétals dérivés du 3,4-dihydro-2H-pyrane, les dérivés acylés ou aryliques de warfarine et l'oxime de 4-hydroxy-3-[1-(4-chloro-phényl)-3-oxo-butyl]-2H-1-benzopyrane-2-one.

9. Procédé conforme à la revendication 1, dans lequel on déduit le génotype CYP4F2 grâce à une analyse du site rs2108622.

10. Procédé conforme à la revendication 1, dans lequel on déduit le génotype CYP4F2 grâce à une analyse d'un site SNP choisi parmi les sites rs3093114, rs3093106 et rs3093105.
